# EUROPEAN PATENT SPECIFICATION

(11) **EP 2 701 691 B1**
(45) Date of publication and mention of the grant of the patent: **13.04.2016**
(21) Application number: 12717735.0
(22) Date of filing: 29.04.2012
(51) Int. Cl.: A61K 31/05, A61K 31/07, A61K 31/198, A61K 31/352, A61K 31/355, A61K 31/59, A61K 31/702, A61K 31/715, A61K 38/43, A61K 38/06, A61K 45/06, A61K 38/48, A61K 38/47, A61K 38/46

(54) **COMPOSITION FOR PREVENTION AND TREATMENT OF RHEUMATOID ARTHRITIS**
ZUSAMMENSETZUNG ZUR PRÄVENTION UND BEHANDLUNG VON RHEUMATOIDER ARTHRITIS
COMPOSITION POUR LA PREVENTION ET LE TRAITEMENT DE LA POLYARTHRITE RHUMATOIDE

(30) Priority: 29.04.2011 EP 11164261
(43) Date of publication of application: 05.03.2014
(73) Proprietor: Biotechnobel SA, 1190 Bruxelles (BE)
(72) Inventor: BATHAEI, Seyed Saied, 1190 Bruxelles (BE)
(74) Representative: Callewaert, Raf
(86) International application number: PCT/EP2012/057875
(87) International publication number: WO 2012/146773

(56) References cited:
- WO-A1-2009/086614
- US-A1- 2003 143 292
- US-A1- 2004 253 296

## Description

The present invention relates to a pharmaceutical or neutraceutical composition for prevention and treatment of rheumatoid arthritis (RA) and for providing beneficial health effects in relation to rheumatoid arthritis.

Arthritis is a degenerative condition that affects the joints causing severe pain and greatly reducing mobility. In severe cases and if neglected the effects can be debilitating.

Rheumatoid arthritis (RA) is a chronic, systemic inflammatory disorder that may affect many tissues and organs, but principally attacks synovial joints ⁽¹⁻⁴⁾.

Arthritis of joints known as synovitis is inflammation of the synovial membrane that lines joints and tendon sheaths. Joints become swollen, tender and warm, and stiffness limits their movement. With time RA nearly always affects multiple joints resulting in so called polyarthritis. Most commonly small joints of the hands, feet and cervical spine are involved, but larger joints like the shoulder and knee can also be involved. Synovitis can lead to tethering of tissue with loss of movement and erosion of the joint surface causing deformity and loss of function ⁽⁵⁻⁶⁾.

Rheumatoid arthritis typically manifests with signs of inflammation, with the affected joints being swollen, warm, painful and stiff, particularly early in the morning on waking or following prolonged inactivity. Increased stiffness early in the morning is often a prominent feature of the disease and typically lasts for more than an hour. Gentle movements may relieve symptoms in early stages of the disease. These signs help distinguish rheumatoid from osteoarthritis or "wear-and-tear" arthritis. In arthritis of non-inflammatory causes, signs of inflammation and early morning stiffness are less prominent with stiffness typically less than 1 hour, and movements induce pain caused by mechanical arthritis ⁽⁷⁾.

Osteoarthritis is often treated by the administration of glucosarnine as described in e.g. US patent applications US2003/0143292A1 and US2004/0253296A1. This is only intended to reduce pain and discomfort in the synovial joints. Rheumatoid arthritis (RA) in contrast is a systemic inflammatory disorder, which not only affects the joints.

International patent application WO2009/086614A1 describes a symbiotic food/feed composition for the well-being of humans and animals comprising probiotic, prebiotic, essential fatty acid, vitamin, enzyme, mineral, phytochemical, nutritional and nutraceutical components. However, it does not disclose a composition for preventing or treating rheumatoid arthritis.

There is no known cure for rheumatoid arthritis, but many different types of treatment can alleviate symptoms and/or modify the disease process. The goal of treatment is twofold: alleviating the current symptoms, and preventing the future destruction of the joints with the resulting handicap if the disease is left unchecked. These two goals may not always coincide: while pain relievers may achieve the first goal, they do not have any impact on the long-term consequences ⁽⁸⁾.

Most treatments focus on pain relief. Analgesia (painkillers) and antiinflammatory drugs, including steroids, are used to suppress the symptoms, while disease-modifying antirheumatic drugs (DMARDs) are required to inhibit or halt the underlying immune process and prevent long-term damage ⁽⁹⁻¹⁰⁾. There are Non Steroidal Anti-Inflammatory Drugs (NSAIDS) such as aspirin, and SAIDS such as cortisone. The NSAID's inhibit cyclooxygenase, which is the enzyme necessary to the synthesis of the series 2 prostaglandins, which decrease inflammatory and proinflammatory state. The SAID's do the same at a higher biochemical level. This takes away signalling functions of the body and is not beneficial long term. It worsens a person's health in many cases.

As a conclusion, existing treatments are mostly not inducing a healing effect, but rather only mask symptoms or at the best slow down the progress of the disease.

The present invention aims to provide a pharmaceutical or nutraceutical composition for prevention and/or treatment of rheumatoid arthritis. The invention aims to reduce and prevent the chronic inflammation, which is at the origin of rheumatoid arthritis, and is based on a synergetic effect of certain dietary fibres in combination with antioxidants, enzymes, amino acids, minerals and/or vitamins.

To this end, the present invention provides a pharmaceutical composition for preventing or treating rheumatoid arthritis containing a pharmaceutically effective amount of at least prebiotic dietary fibres, antioxidants comprising polyphenols, enzymes comprising at least an amylolytic enzyme, at least a liposoluble vitamin, and glutamine as set out in the annexed claims.

According to the invention these compounds may be offered as food supplement, functional food ingredients or as pharmaceutical ingredients in order to prepare a composition for oral administration for preventing and/or treating rheumatoid arthritis.

The core of this invention is that one recognizes the multidimensional causes of arthritis in order to offer a multiple approach, which balances step by step the different biochemical processes concerned, and, hence, improves the condition of specific types of cells. These specific cells are enterocytes, colonocytes, hepatocyts, immune cells, osteoblasts and the gut flora. The different ingredients of the composition according to the invention are focused to balance and optimize the metabolic and biochemical functions of these cells. Only if sufficient essential elements are present, a synergy emerges and the healing process starts.

The composition according to the invention contains preferably at least four groups of ingredients: prebiotic dietary fibres, enzymes, antioxidants and optionally also a blend of minerals, vitamins and amino acids. Further, additional ingredients may be added, which improve the effect of other ingredients and may consist of enzymes.

As such, the composition according to the invention may contain at least 20 weight %, preferably at least 60 weight %, more preferably at least 80 weight % of prebiotic dietary fibre, the total of the composition being 100 weight %. Furthermore, the composition according to the invention may contain at least 1 weight %, preferably at least 2 weight %, more preferably at least 8 weight % of antioxidants including polyphenols, the total of the composition being 100 weight %. The composition also contains at least an amylolytic enzyme and at least a liposoluble vitamin. Preferably, the composition contains at least 1 weight %, preferably at least 2 weight % of enzymes and at least 1 weight %, preferably at least 2 weight % of a blend of vitamins, dietary minerals and/or amino acids, the total of the composition being 100 weight %.

A first important element in the composition according to this invention is to offer prebiotic dietary fibres.

Dietary fibres are an important part of a healthy and balanced nutrition and are important for its role in the health of the colon and the small intestine cells, i.e. colonocytes and enterocytes. Dietary fibres may prevent damage of them.

Dietary fibres are generally regarded as the indigestible portion of plant or vegetable foods. They are carbohydrates, more specifically non-starch poly-and oligosaccharides, and lignin, primarily derived from plant cell walls that cannot be hydrolyzed by human digestive enzymes. However, they often can be fermented by intestinal bacteria to produce hydrogen, methane, carbon dioxide, water and short chain fatty acids (SCFA).

Its function differs in the small and large intestine. Its primary function in the small intestine is to enhance viscosity, while in the large intestine, it is to serve as a substrate for the gut flora in order to produce the short chain fatty acids.

The short chain fatty acids are very beneficial for the development of the intestinal cells and thus form one of the most important benefits of dietary fibres.

Short chain fatty acids are by-products of the fermentation of soluble dietary fibres in the colon. SCFA are involved in numerous physiological processes promoting health. Amongst others they stabilize blood glucose levels by acting on pancreatic insulin release and liver control of glycogen. They provide nourishment of colonocytes, in particularly butyrate. They suppress cholesterol synthesis by the liver and reduce blood levels of LDL cholesterol and triglycerides responsible for atherosclerosis. They lower colonic pH, stimulate the production of T-helper cells, antibodies, etc. and improve intestinal barrier properties.

In this invention soluble dietary fibres are, preferably, used as well in form of large chains, i.e. polysaccharides, as in form of short chains, i.e. oligosaccharides. When soluble fibres are consumed, the undigested portion serves as substrate for the gut flora. Depending on the type of soluble fibre, different bacterial groups are stimulated. Therefore the present invention proposes to use preferential at least two types of fibre in order to obtain an optimal result. Several clinical studies have shown that administering oligosaccharides such as fructo-oligosaccharides (FOS), galacto-oligosaccharides (GOS or GaOS), gluco-oligosaccharides (GOS or GluOS) or xylo-oligosaccharides (XOS) can increase the number of friendly or beneficial bacteria while reducing the population of harmful bacteria.

Several dietary fibres, such as the oligosaccharides discussed, have also been defined as prebiotics. For a food ingredient to be classified as a prebiotic it must fulfil the following criteria: (i) neither be hydrolysed, nor absorbed in the upper gastrointestinal tract, (*ii*) be selectively fermented by one or a limited number of bacteria potentially beneficial to the colon, and (*iii*) be able to alter the colonic micro flora towards a healthier composition, by increasing friendly bacteria and reducing harmful or putrefactive bacteria.

Since dietary fibres provide such a broad range of nutritional and health benefits, they are added to many food products and are provided as separate food supplements or prebiotic compositions.

WO 2007/050656 A2 for example describes a food supplement based on dietary fibres. The food supplement is a fibre formulation comprising in its primary embodiment partially-hydrolyzed guar gum (PHGG) and fructooligosaccharides (FOS), wherein the dietary fibre formulation exhibits a prebiotic potential greater than a prebiotic potential of PHGG and FOS individually.

WO 2004/052121 describes prebiotic compositions comprising soluble fibres, in particular fructo-oligosaccharides (FOS) and galacto-oligosaccharides (GOS) and their use in the treatment of prevention of gastrointestinal tract disorders, such as inflammatory bowel disease (IBD) diarrhoea and constipation.

US2008/0261919A1 describes synergistic compositions comprising prebiotic components selected from a large group comprising amongst many others modified or unmodified starch and partial hydrolysates thereof, fructo-oligosaccharides, trans-galacto-oligosaccharides, xylo-oligosaccharides (XOS), betaglycan and partial hydrolysates thereof, end if desired other plant extracts, mineral components, vitamins and additives.

All of the previously described compositions relate to prebiotic mixtures of dietary fibres and provide some sort of beneficial health effects by stimulating the micro-organisms predominantly present in the large intestine. However, none of the compositions manages to stimulate the micro bacterial gut flora in an optimal manner.

WO 2004/052121 describes a mixture of fructo-oligosaccharides (FOS) and galacto-oligosaccharides (GOS) and their beneficial effects, as well as the synergy between these two prebiotic fibres. In other described embodiments, also other prebiotic oligosaccharides such as xylo-oligosaccharides (XOS) are comprised in the composition.

These two publications thus describe compositions which comprise more prebiotic compounds, and will thus provide a more varied nutrition for beneficial microbial bacteria. A healthy microbial flora helps to restore the barrier function of the colon wall.

In a possible embodiment of the present invention, short chain fatty acids (SCFA) can be added. SCFA's can replace the fibres in the composition or be added to the fibres in the composition.

Preferably, a combination of fructo-oligosaccharides (FOS), xylo-oligosaccharides (XOS) and gluco-oligosaccharides (GOS) are used in the composition. The invention is however not restricted to the mentioned types of dietary fibres. All other dietary fibres considered suitable by the person skilled in the art can also be used in the composition.

A second important element in the composition according to this invention is to offer antioxidants. Chronic inflammation creates oxidative stress which in turn creates inflammation which then creates oxidative stress. The use of antioxidants in the composition according to the invention breaks this spiral.

In normal conditions, the body forms reactive oxygen species (ROS) that are called primary free radicals and protects itself against it by the production of primary antioxidants such as superoxide dismutase (SOD), catalase, peroxidase and other specialized enzymes. Hence, primary antioxidants are antioxidants for neutralising primary free radicals such as the hydroxyl radical (⁻OH) and the superoxide anion radical (O₂⁻).

However, if too much primary free radicals are present, they will not be eliminated and will form secondary free radicals in a chain reaction. A typical example is the peroxidation of lipids, causing artherosclerosis.

Defence against these secondary free radicals can come from secondary antioxidants such as vitamin A, vitamin C (ascorbic acid), vitamin E (tocopherols, tocotrienols), polyphenolic antioxidants (resveratrol, flavonoids), carotenoids (lycopene, carotenes, lutein), glutathione, coenzyme Q10 (ubiquinon), selenium, zinc and/or copper.

Hence, secondary antioxidants are antioxidant for neutralising organic molecules, such as proteins and fats, which have reacted with a very reactive superoxide and received a free radical. This will prevent that free radicals will propagate and create more damage. Free radicals that interact with other target molecules gain a stable configuration of electrons and will convert the target molecules into radicals, which are called here secondary free radicals. So a chain reaction begins that will propagate until two radicals meet each other and each contributes its unpaired electron to form a covalent bond linking the two.

Examples of interesting secondary antioxidants that can be used in a composition according to the invention are: flavonoids such as quercetin and epicatechin, obtainable from *Camellia sinensis* and/or *Citrus aurantium;* polyphenols and anthocyanin obtainable from *Punica granatum, Curcuma longa* and/or *Crocus sativus.*

Administrating secondary antioxidants only will not take away the source of their creation, and hence may have a limited effect. Preferably, the composition comprises a combination of primary antioxidants and secondary antioxidants.

A separate class of molecules, which may also be used in a composition according to the invention, are identified herein as indirect antioxidants. These indirect antioxidants consist of polyunsaturated fatty acids, which help to prevent formation of free radicals. Research shows that omega-3 fatty acids reduce inflammation. A reduction of inflammation means less creation of free radicals and hence an indirect protection against oxidation. Clinical evidence suggests that eicosapentaenoic acid (EPA) and docosahexaenoic acid (DHA), i.e. the two omega-3 fatty acids found in fish oil, or α-linolenic acid (ALA), obtained from e.g. flaxseed, are fatty acids which are beneficial in this composition.

As such, the antioxidants in the composition according to the invention may be a combination of at least or one primary antioxidant and/or at least one secondary antioxidant and/or at least one indirect antioxidant.

Preferably, the composition according to the invention also contains at least a third key element, namely enzymes that help the digestive system.

Amylolytic, lipolytic and proteolytic enzymes help in reducing the overload on the digestive system. Proteolytic enzymes such as bromelain, nattokinase, protease, serapeptidase or papain can also help to reduce lesions and inflammation. Proteolytic enzymes may be added to reduce inflammation and for their fibrinolytic action.

A fourth optional element in the composition according to this invention is a blend of minerals, vitamins and amino acids, which provide the necessary cofactors and coenzymes in order to optimize the action of others ingredients.

As such, preferably, the prebiotic dietary fibres are supplemented with elements which are stimulating the restoration and renewal of the colonocyts. Examples of these stimulating factors are amino acids such as glutamine, lysine, methionine, cysteine, vitamins such as vitamin A, C, D, E, B6, B9, B12, and dietary minerals such as zinc, selenium and copper. Liposoluble vitamins play an important role. Examples of liposoluble vitamins are vitamin A, vitamin E, vitamin K and vitamin D.

Glutamine, in particular, is a very interesting amino acid in the composition according to the invention since it helps in the restoration of colonocyts.

Synergy of different ingredients of the composition only emerges when sufficient ingredients of the different elements are present at the same time. Hence, there is some freedom in the choice of ingredients. Many ingredients are beneficial for more than one type of cell. The synergy of the elements to treat chronic inflammation, which is the root cause of rheumatoid arthritis is essential. We have found that osteoarthritis is often a consequence of rheumatoid arthritis, and, hence, this composition will also reduce the symptoms of osteoarthritis. This effect is obtained without needing to provide aminosaccharides such as glucosamine in the composition according to the invention.

The four elements for the treatment can be offered as separate compositions or combined. For the optimal treatment of rheumatoid arthritis, all four elements are needed.

According to the invention, the composition may contain 20-90 weight %, of dietary fibres, 1-20 weight % of antioxidants, 1-20 weight % of enzymes, 1-20 % of vitamins and optionally minerals, and 1-40 % of optionally amino acids, wherein the total of the composition being 100 weight %. Table 5 gives an example of preferred ranges of ingredients of a possible composition according to the inventions.

Tables 6, 7 and 8 are examples of specific compositions according to embodiments of the inventions. It should be noted that variations of concentrations of the different ingredients by 10 to 20 % or more will still result in compositions that are sufficiently effective and are within the scope of the present invention. Treatment with a daily dose of 2,5 g to 7,5 g of these compositions is recommended. It should be noted that both higher and lower doses could be considered to be useful.

In a preferred embodiment of the present invention, the composition is provided in a substantially dry formulation.

In a dry formulation, the composition will be preserved longer and potential enzymes comprised in the composition will not be in an active state. This will eliminate the risk that the enzymes will start digesting and cleaving the fibres before the composition enters the human gastrointestinal tract.

The composition is thus preferably provided in a formulation such as a powder, possibly compressed in e.g. a tablet or pill or encapsulated in e.g. a gelatine capsule. Fibres, antioxidants, enzymes and vitamins can be part of separate of combined compositions.

### Clinical trial:

Rheumatoid arthritis (RA) is an inflammatory form of arthritis that causes joint pain and damage. RA attacks the lining of the joints (synovium), causing swelling that can result in aching and throbbing, and eventually deformity. It is likely that RA occurs as a result of a complex combination of factors, including a person's genes; lifestyle choices, such as smoking and diet; and things in a person's environment, including bacteria or viruses.

Musculoskeletal complications are frequent and well-recognized manifestations in inflammatory bowel disease (IBD), and affect up to 33% of patients with IBD. The strong link between the bowel and the osteo-articular system is suggested by many clinical and experimental observations ⁽¹¹⁻¹³⁾.

The present study investigates the hypothesis that disequilibrium of the bacteria living in a person's intestinal tract is responsible, at least in part, for the development of rheumatoid arthritis.

Rheumatoid arthritis (RA) biomarkers, including rheumatoid factor (RF), erythrocyte sedimentation rate (ESR), anti-CCP antibodies, high sensitivity C-reactive protein (US-CRP) and biomarkers of bone and cartilage turnover, have been used. The biomarkers have been evaluated before treatment and at 3 and 6 months after treatment, in order to determine whether changes are associated with the treatment.

In this study treatment of test persons with a test composition of 5 g per day during at least 6 months has been examined. Results are shown in table 1.

The impact of oral ingestion of a test composition on clinical variables associated with inflammation and on general health of patients with RA has been evaluated.

A first test composition containing only a prebiotic supplement, as shown in table 2, has been used in a first group of test persons.

A second test composition containing a prebiotic supplement with a blend of antioxidants, as shown in table 3, has been used in a second group of test persons.

A third test composition containing a prebiotic supplement with a blend of enzymes and antioxidants, as shown in table 4, has been used in a third group of test persons.

A fourth test composition containing a prebiotic supplement with a blend of enzymes, antioxidants and a complex of vitamins, minerals and amino acids, as shown in table 5, has been used in a fourth group of test persons.

All test persons were randomly assigned to receive test compositions or a placebo for 6 months.

### Results:

The study showed that after six months the first test composition resulted in a moderate reduction in pain levels, but did not result in any clinically significant improvement in disease activity when assessing the study group as a whole.

In second group of test persons, it was observed that the dietary supplementation with prebiotic fibres and antioxidants resulted in a significant reduction in the rheumatoid arthritis (RA) biomarkers.

In the third group of test persons, the dietary supplementation with prebiotic fibres, a blend of enzymes and antioxidants resulted in a significant reduction in the rheumatoid arthritis (RA) biomarkers that was significant higher than in the second group of test persons.

Finally, in the fourth group of test persons, the dietary supplementation with prebiotic fibres, a blend of enzymes, antioxidants and a complex of vitamins, minerals and amino acids resulted in a significant higher reduction in the rheumatoid arthritis (RA) biomarkers than in the third group of test persons. All persons of the group showed also significant reductions in pain levels.

Hence, a clear cascade of improvements of symptoms and biomarkers can be observed between the different compositions due to the synergy of different ingredients of the composition, i.e. prebiotic dietary fibres, antioxidants comprising polyphenols, enzymes comprising an amylolytic enzyme, amino acids including glutamine, and further elements such as vitamins, in particular liposoluble vitamins.

**Table 1: Test results of a clinical trial using four test compositions of Tables 2 to 5.**

| **Test group** | Symptoms improvement** | Biomarkers improvement*** |
|---|---|---|
| **Composition** | | |
| **Group 1 (n* =25)** | + | - |
| First test composition of prebiotics (Table 2) | | |
| **Group 2 (n=22)** | ++ | + |
| Second composition of prebiotics and antioxidants (Table 3) | | |
| **Group 3 (n=23)** | ++ | ++ |
| Third test composition of prebiotics, enzymes and antioxidants (Table 4) | | |
| **Group 4 (n=26)** | +++ | +++ |
| Fourth test composition of prebiotics, enzymes, antioxidants, vitamins, minerals and amino acids (Table 5) | | |
| **Placebo (n=25)** | - | - |

| | | |
|---|---|---|
| * n, number of test persons; ** reductions in pain levels *** reduction of rheumatoid arthritis (RA) biomarkers, including rheumatoid factor (RF), erythrocyte sedimentation rate (ESR), anti-CCP antibodies, high sensitivity C-reactive protein (HS-CRP) and biomarkers of bone and cartilage turnover; + significant improvement ("+++" indicates a significant higher improvement than "++", which in turn indicates a significant higher improvement than "+"); - no effect. | | |

**Table 2: First test composition of prebiotics.**

| **Ingredients** | **Portion (+/- 5 g/day)** | **% weight** |
|---|---|---|
| Dietary fibres (FOS, GOS, XOS) | 5,000 g | 100,00 % |
| TOTAL | **5,000 g** | **100 %** |

**Table 3: Second test composition of prebiotics and antioxidants.**

| **Ingredients** | **Portion (+/- 5 g/day)** | **% weight** | **Preferred range (minimum - maximum)** | |
|---|---|---|---|---|
| Dietary fibres (FOS, GOS, XOS) | 4,900 g | 98,00 % | 4,00 - 4,95 g | 80,00 - 99,00 % |
| Antioxidants (polyphenols) | 0,100 g | 2,00 % | 0,05 - 1,00 g | 1,00 - 20,00 % |
| **TOTAL** | **5,000 g** | **100 %** | **5,000 g** | **100 %** |

**Table 4: Third test composition of prebiotics, antioxidants and enzymes.**

| **Ingredients** | **Portion (+/- 5 g/day)** | **% weight** | **Preferred range (minimum - maximum)** | |
|---|---|---|---|---|
| Enzymes (xylanase, pectinase, lactase, amylase, lipase, proteases) | 0,100 g | 2,00% | 0,05 - 1,00 g | 1,00 - 20,00 % |
| Dietary fibres (FOS, GOS, XOS) | 4,800 g | 96,00 % | 3,00 - 4,80 g | 60,00 - 96,00 % |
| Antioxidants (polyphenols) | 0,100 g | 2,00 % | 0,45 - 1,00 g | 1,00 - 20,00 % |
| **TOTAL** | **5,000 g** | **100 %** | **5,000 g** | **100 %** |

**Table 5: Fourth test composition of prebiotics, antioxidants, enzymes, vitamins, minerals and amino acids.**

| **Ingredients** | **Portion (+/- 5 g/day)** | **% weight** | **Preferred range (minimum - maximum)** | |
|---|---|---|---|---|
| Enzymes (xylanase, pectinase, lactase, amylase, lipase, protease) | 0,100 g | 2,00 % | 0,05-1,00 g | 1,00 - 20,00 % |
| Dietary fibres (FOS, GOS, XOS) | 4,025 g | 80,50 % | 1,00 - 4,50 g | 20,00 - 90,00 % |
| Glutathione | 0,250 g | 5,00 % | 0,10 - 0,40 g | 2,00 - 8,00 % |
| Omega 3 (EPA, DHA) | 0,150 g | 3,00 % | 0,00 - 2,00 g | 0,00 - 40,00 % |
| Vitamins (A, C, D, E, B6, B9) and minerals (zinc, copper, selenium) | 0,075 g | 1,50 % | 0,05 - 1,00 g | 1,00 - 20,00 % |
| Antioxidants (polyphenols) | 0,100 g | 2,00 % | 0,05 - 1,00 g | 1,00 - 20,00 % |
| Calcium and vitamin D3 and K | 0,100 g | 2,00 % | 0,05 - 1,00 g | 1,00 - 20,00 % |
| Amino acids (glutamine, lysine, methionine, cysteine) | 0,200 g | 4,00 % | 0,05 - 2,00 g | 2,00 - 40,00 % |
| **TOTAL** | **5,000 g** | **100 %** | **5,000 g** | **100 %** |

**Table 6: Example of a specific composition according to an embodiment of the invention.**

| **Ingredients** | **Portion (+/- 5 g/day)** | **% weight** |
|---|---|---|
| Xylanase | 2,5 mg | 0,05 |
| Amylase | 5,0 mg | 0,10 |
| Protease | 5,0 mg | 0,10 |
| Omega 3 | 100,0 mg | 2,00 |
| Inuline | 1,075 g | 21,50 |
| Dextrine | 2,123 g | 42,45 |
| Bran of barley | 1,075 g | 21,50 |
| Glutamine | 250,0 mg | 5,00 |
| Vitamin D3 and K | 25,0 mg | 0,50 |
| Pomegranate extract (polyphenols) | 65,0 mg | 1,30 |
| Citrus extract (polyphenols) | 50,0 mg | 1,00 |
| Cysteine | 200,0 mg | 4,00 |
| Lysine | 25,0 mg | 0,50 |
| **TOTAL** | **5,0 g** | **100 %** |

**Table 7: Example of a specific composition according to another embodiment of the invention.**

| **Ingredients** | **Portion (+/- 5 g/day)** | **% weight** |
|---|---|---|
| Xylanase | 2,5 mg | 0,05 |
| Amylase | 5,0 mg | 0,10 |
| Protease | 5,0 mg | 0,10 |
| Omega 3 | 100,0 mg | 2,00 |
| Inuline | 1,075 g | 21,50 |
| Dextrine | 2,120 g | 42,40 |
| Bran of barley | 1,075 g | 21,50 |
| Glutamine | 250,0 mg | 5,00 |
| Vitamin D3 and K | 25,0 mg | 0,50 |
| Vitamin A | 2,5 mg | 0,05 |
| Pomegranate extract (polyphenols) | 65,0 mg | 1,30 |
| Citrus extract (polyphenols) | 50,0 mg | 1,00 |
| Cysteine | 200,0 mg | 4,00 |
| Lysine | 25,0 mg | 0,50 |
| **TOTAL** | **5,0 g** | **100 %** |

**Table 8: Example of a specific composition according to still another embodiment of the invention.**

| **Ingredients** | **Portion (+/- 5 g/day)** | **% weight** |
|---|---|---|
| Xylanase | 2,5 mg | 0,05 |
| Amylase | 5,0 mg | 0,10 |
| Protease | 5,0 mg | 0,10 |
| Omega 3 | 100,0 mg | 2,00 |
| Inuline | 1,075 g | 21,50 |
| Dextrine | 2,120 g | 42,40 |
| Bran of barley | 1,075 g | 21,50 |
| Glutamine | 250,0 mg | 5,00 |
| Vitamin D3 and K | 25,0 mg | 0,50 |
| Vitamin A | 2,5 mg | 0,05 |
| Pomegranate extract (polyphenols) | 62,0 mg | 1,24 |
| Citrus extract (polyphenols) | 50,0 mg | 1,00 |
| Catalase | 1,0 mg | 0,02 |
| Superoxide dismutase (SOD) | 2,0 mg | 0,04 |
| Cysteine | 200,0 mg | 4,00 |
| Lysine | 25,0 mg | 0,50 |
| **TOTAL** | **5,0 g** | **100 %** |

### REFERENCES

1- Furfaro N.New therapeutic options in rheumatoid arthritis: a focus on interleukin-6 blockade. J Infus Nurs. 2011 Mar-Apr;34(2):107-15.
2- Trieb K, Hofstaetter SG. Treatment strategies in surgery for rheumatoid arthritis. Eur J Radiol. 2009 Aug;71(2):204-10.
3-Thomson S, Udalova IA. Quantitative measurement of cytokine expression in synoviocytes derived from rheumatoid arthritis patients. Methods Mol Biol. 2009;512:233-48*.*
4- Wang Q, Ma Y, Liu D, Zhang L, Wei W. The Roles of B Cells and Their Interactions with Fibroblast-Like Synoviocytes in the Pathogenesis of Rheumatoid Arthritis. Int Arch Allergy Immunol. 2011 Feb 2; 155(3):205-211*.*
5- Woolley DE, Crossley MJ, Evanson JM. Collagenase at sites of cartilage erosion in the rheumatoid joint. Arthritis Rheum. 1977 Jul-Aug;20(6):1231-9.
6- Jalava S, Reunanen K. Healing of erosions in rheumatoid arthritis. Scand J Rheumatol 1982; 11(2):92-100*.*
7- Jijith Krishnan,Document on Rhuematoid arthritis". Pn.lifehugger.com. http://pn.lifehugger.com/doc/459/An_approach_to_Earl_ Arthritis. Retrieved March 3, 2011.
8- Saag KG, Teng GG, Patkar NM, Anuntiyo J, et al. American College of Rheumatology 2008 recommendations for the use of nonbiologic and biologic disease-modifying antirheumatic drugs in rheumatoid arthritis. Arthritis Rheum. 2008 Jun 15;59(6):762-84.
9- Majithia V, Geraci SA. Rheumatoid arthritis: diagnosis and management. Am J Med. 2007 Nov;120(11):936-9.
10-Landré-Beauvais AJ. The first description of rheumatoid arthritis. Unabridged text of the doctoral dissertation presented in 1800. Joint Bone Spine. 2001 Mar;68(2):130-43.
11- Tatiana Sofia Rodriguez-Reyna, Cynthia Martinez-Reyes, Jesús Kazúo Yamamoto-Furusho. Rheumatic manifestations of inflammatory bowel disease. World J Gastroenterol 2009 November 28; 15(44): 5517-5524.
12- Severijnen AJ, van Kleef R, Hazenberg MP, van de Merwe JP. Cell wall fragments from major residents of the human intestinal flora induce chronic arthritis in rats. J Rheumatol 1989;16:1061-8.
13- Zhang X, Rimpilainen M, Hoffmann B, Simelyte E, Aho H, Toivanen P. Experimental chronic arthritis and granulomatous inflammation induced by bifidobacterium cell walls. Scand J Immunol 2001;54:171-9.

## Claims

1. A pharmaceutical composition for use in the treatment of rheumatoid arthritis containing a pharmaceutically effective amount of at least
(*i*) prebiotic dietary fibres;
(*ii*) antioxidants comprising polyphenols;
(*iii*) enzymes comprising at least an amylolytic enzyme;
(*iv*) liposoluble vitamins comprising vitamin D and/or vitamin A; and
(v) amino acids comprising at least glutamine.

2. Composition for use in the treatment of rheumatoid arthritis according to claim 1, whereby said prebiotic dietary fibres contain at least xylo-oligosaccharides (XOS) and gluco-oligosaccharide (GuOS).

3. Composition for use in the treatment of rheumatoid arthritis according to claim 1 or 2, whereby the prebiotic dietary fibre contains at least three different oligosaccharides selected from the group consisting of fructo-oligosaccharides (FOS), xylo-oligosaccharides (XOS), gluco-oligosaccharide (GuOS) and/or galacto-oligosaccharide (GaOS).

4. Composition for use in the treatment of rheumatoid arthritis according to any of the claims 1 to 3, whereby the prebiotic dietary fibre contains oligosaccharides and/or polysaccharides selected from the group consisting of fructo-oligosaccharides (FOS), xylo-oligosaccharides (XOS), gluco-oligosaccharide (GuOS), galacto-oligosaccharide (GaOS), polydextrose, inulin, dextrin or a combination thereof.

5. Composition for use in the treatment of rheumatoid arthritis according to any of the claims 1 to 4, whereby the antioxidants are a combination of
- at least one primary antioxidant selected from the group consisting of superoxide dismutase, catalase and/or peroxidase, or a combination thereof, and
- at least one secondary antioxidant selected from the group consisting of
flavonoids such as quercetin and epicatechin obtainable from *Camellia sinensis* and/or *Citrus aurantium,*
polyphenols and anthocyanin obtainable from *Punica granatum*, *Curcuma longa* and/or *Crocus sativus,*
vitamin A, vitamin C, vitamin E, polyphenolic antioxidants, carotenoids, glutathione, coenzyme Q10, selenium, zinc, and/or copper, or a combination thereof.

6. Composition for use in the treatment of rheumatoid arthritis according to any of the claims 1 to 5, whereby the antioxidants contain at least one polyunsaturated fatty acid.

7. Composition for use in the treatment of rheumatoid arthritis according to any of the claims 1 to 6, whereby the antioxidants contain polyphenol, flavonoid and/or anthocyanin obtained from *Punica granatum, Curcuma longa, Citrus aurantium, Crucus sativum*, cranberry, or a combination thereof.

8. Composition for use in the treatment of rheumatoid arthritis according to any of the claims 1 to 7, whereby the enzymes further contain enzymes selected from the groups of proteolytic and/or lipolitic enzymes.

9. Composition for use in the treatment of rheumatoid arthritis according to any of the claims 1 to 8, whereby it further contains
- vitamins selected from the group of vitamin A, vitamin C, vitamin D, vitamin E, vitamin B6, vitamin B9 and/or vitamin B 12 or a combination thereof;
- dietary minerals selected from the group of zinc, copper and/or selenium or a combination thereof; and/or
- amino acids selected from the group of lysine, methionine and/or cysteine or a combination thereof.

10. A composition for use in the treatment of rheumatoid arthritis according to any of the claims 1 to 9, whereby it contains
20-90 weight %, of dietary fibres,
1-20 weight % of antioxidants,
1-20 weight % of enzymes,
1-20 weight % of vitamins and optionally dietary minerals,
1-40 weight % of amino acids,
the total of the composition being 100 weight %.

11. A composition for use in the treatment of rheumatoid arthritis according to any of the claims 1 to 10, whereby it contains said prebiotic dietary fibres in an amount of
0-30 weight %, preferably 5-15 weight %, of fructo-oligosaccharides,
0-30 weight %, preferably 5-20 weight %, of gluco-oligosaccharide,
0-40 weight %, preferably 15-30 weight %, of xylo-oligosaccharides,
the total of the composition being 100 weight %.

12. Composition for use in the treatment of rheumatoid arthritis according to any of the preceding claims, whereby it further contains short chain fatty acids in combination with the prebiotic dietary fibre.

13. Composition for use in the treatment of rheumatoid arthritis according to any of the preceding claims, whereby it contains 2 to 10 weight % of glutamine, preferably about 4 to 5 weight %, the total of the composition being 100 weight %.

14. Food supplement for use in the prevention or treatment of rheumatoid arthritis containing a composition according to any of the preceding claims.

15. Functional food for use in the prevention or treatment of rheumatoid arthritis and reduction of the symptoms of osteoarthritis containing a composition according to any of the preceding claims.

## Patentansprüche

1. Eine pharmazeutische Zusammensetzung zur Verwendung in der Behandlung von rheumatoider Arthritis, die eine pharmazeutisch wirksame Menge enthält von mindestens
(i) prebiotischen Ballaststoffen;
(ii) Antioxidantien, die Polyphenole umfassen;
(iii) Enzymen, die mindestens ein amylolytisches Enzym umfassen;
(iv) fettlöslichen Vitaminen, die Vitamin D und/oder Vitamin A umfassen; und
(v) Aminosäuren, die mindestens Glutamin umfassen.

2. Zusammensetzung zur Verwendung in der Behandlung von rheumatoider Arthritis gemäß Anspruch 1, wobei diese prebiotischen Ballaststoffe mindestens Xylooligosaccharide (XOS) und Glukooligosaccharide (GuOS) umfassen.

3. Zusammensetzung zur Verwendung in der Behandlung von rheumatoider Arthritis gemäß Anspruch 1 oder 2, wobei der prebiotische Ballaststoff mindestens drei verschiedene Oligosaccharide umfasst, die ausgewählt werden aus der Gruppe, die besteht aus Fructooligosacchariden (FOS), Xylooligosacchariden (XOS), Glukooligosacchariden (GuOS) und/oder Galactooligosacchariden (GaOS).

4. Zusammensetzung zur Verwendung in der Behandlung von rheumatoider Arthritis gemäß einem der Ansprüche 1 bis 3, wobei der prebiotische Ballaststoff Oligosaccharide und/oder Polysaccharide umfasst, die ausgewählt werden aus der Gruppe, die besteht aus Fructooligosacchariden (FOS), Xylooligosacchariden (XOS), Glukooligosaccharid (GuOS), Galactooligosaccharid (GaOS), Polydextrose, Inulin, Dextrin oder einer Kombination daraus.

5. Zusammensetzung zur Verwendung in der Behandlung von rheumatoider Arthritis gemäß einem der Ansprüche 1 bis 4, wobei die Antioxidantien eine Kombination sind aus
- mindestens einem primären Antioxidans, das ausgewählt wird aus der Gruppe, die besteht aus Superoxiddismutase, Katalase und/oder Peroxidase oder einer Kombination daraus, und
- mindestens einem sekundären Antioxidans, das ausgewählt aus der Gruppe, die besteht aus
Flavonoiden wie Quercetin und Epicatechin, die gewonnen werden aus *Camellia sinensis* und/oder *Citrus aurantium,*
Polyphenolen und Anthocyanen, die gewonnen werden aus *Punica granatum, Curcuma longa* und/oder *Crocus savitus,*
Vitamin A, Vitamin C, Vitamin E, Polyphenol-Antioxidantien, Carotenoiden, Glutathion, Co-Enzym Q10, Selen, Zink und/oder Kupfer oder einer Kombination daraus.

6. Zusammensetzung zur Verwendung in der Behandlung von rheumatoider Arthritis gemäß einem der Ansprüche 1 bis 5, wobei die Antioxidantien mindestens eine mehrfach ungesättigte Fettsäure enthalten.

7. Zusammensetzung zur Verwendung in der Behandlung von rheumatoider Arthritis gemäß einem der Ansprüche 1 bis 6, wobei die Antioxidantien Polyphenol, Flavonoid und/oder Anthocyane enthalten, die gewonnen werden aus *Punica granatum, Curcuma longa, Citrus aurantium, Crucus sativum,* Cranberry oder einer Kombination daraus.

8. Zusammensetzung zur Verwendung in der Behandlung von rheumatoider Arthritis gemäß einem der Ansprüche 1 bis 7, wobei die Enzyme darüber hinaus Enzyme enthalten, die aus den Gruppen der eiweißspaltenden und/oder lipolytischen Enzyme ausgewählt werden.

9. Zusammensetzung zur Verwendung in der Behandlung von rheumatoider Arthritis gemäß einem der Ansprüche 1 bis 8, wobei sie darüber hinaus
- Vitamine enthält, die ausgewählt werden aus der Gruppe bestehend aus Vitamin A, Vitamin C, Vitamin D, Vitamin E, Vitamin B6, Vitamin B9 und/oder Vitamin B12 oder einer Kombination daraus;
- Spurenelemente enthält, die ausgewählt werden aus der Gruppe bestehend aus Zink, Kupfer und/oder Selen oder einer Kombination daraus; und/oder
- Aminosäuren enthält, die ausgewählt werden aus der Gruppe bestehend aus Lysin, Methionin und/oder Cystein oder einer Kombination daraus.

10. Eine Zusammensetzung zur Verwendung in der Behandlung von rheumatoider Arthritis gemäß einem der Ansprüche 1 bis 9, wobei sie Folgendes enthält:
20-90 Gewichts-% an Ballaststoffen,
1-20 Gewichts-% an Antioxidantien,
1-20 Gewichts-% an Enzymen,
1-20 Gewichts-% an Vitaminen und optional Spurenelementen,
1-40 Gewichts-% an Aminosäuren,
so dass die Summe der Zusammensetzung 100 Gewichts-% beträgt.

11. Eine Zusammensetzung zur Verwendung in der Behandlung von rheumatoider Arthritis gemäß einem der Ansprüche 1 bis 10, wobei sie diese prebiotischen Ballaststoffe in folgenden Mengen enthält:
0-30 Gewichts-%, bevorzugt 5-15 Gewichts-%, an Fructooligosacchariden,
0-30 Gewichts-%, bevorzugt 5-20 Gewichts-%, an Glucooligosaccharid,
0-40 Gewichts-%, bevorzugt 15-30 Gewichts-%, an Xylooligosacchariden,
so dass die Summe der Zusammensetzung 100 Gewichts-% beträgt.

12. Zusammensetzung zur Verwendung in der Behandlung von rheumatoider Arthritis gemäß einem der voranstehenden Ansprüche, wobei sie darüber hinaus kurzkettige Fettsäuren in Kombination mit dem prebiotischen Ballaststoff enthält.

13. Zusammensetzung zur Verwendung in der Behandlung von rheumatoider Arthritis gemäß einem der voranstehenden Ansprüche, wobei sie 2 bis 10 Gewichts-% an Glutamin, bevorzugt etwa 4 bis 5 Gewichts-%, enthält und die Summe der Zusammensetzung 100 Gewichts-% beträgt.

14. Nahrungsergänzung zur Verwendung in der Prävention oder Behandlung von rheumatoider Arthritis, die eine Zusammensetzung gemäß einem der voranstehenden Ansprüche enthält.

15. Funktionelle Lebensmittel zur Verwendung in der Prävention oder Behandlung von rheumatoider Arthritis und Verringerung der Symptome von Osteoarthritis, die eine Zusammensetzung gemäß einem der voranstehenden Ansprüche enthält.

## Revendications

1. Composition pharmaceutique destiné à être utilisé dans le traitement de la polyarthrite rhumatoïde, contenant une quantité pharmaceutiquement efficace d'au moins
(*i*) des fibres alimentaires prébiotiques;
(*ii*) des antioxydants comprenant des polyphénols;
(*iii*) des enzymes comprenant au moins une enzyme amylolytique;
(*iv*) des vitamines liposolubles comprenant de la vitamine D et/ou de la vitamine A; et
(*v*) des acides aminés comprenant au moins de la glutamine.

2. Composition destiné à être utilisé dans le traitement de la polyarthrite rhumatoïde selon la revendication 1, dans laquelle lesdites fibres alimentaires prébiotiques comprennent au moins des xylo-oligosaccharides (XOS) et des gluco-oligosaccharides (GuOS).

3. Composition destiné à être utilisé dans le traitement de la polyarthrite rhumatoïde selon la revendication 1 ou 2, dans laquelle les fibres alimentaires prébiotiques comprennent au moins trois oligosaccharides différents choisis dans le groupe constitué des fructo-oligosaccharides (FOS), des xylo-oligosaccharides (XOS), des gluco-oligosaccharides (GuOS) et/ou des galacto-oligosaccharides (GaOS).

4. Composition destiné à être utilisé dans le traitement de la polyarthrite rhumatoïde selon l'une quelconque des revendications 1 à 3, dans laquelle les fibres alimentaires prébiotiques comprennent des oligosaccharides et/ou des polysaccharides choisis dans le groupe constitué des fructo-oligosaccharides (FOS), des xylo-oligosaccharides (XOS), des gluco-oligosaccharides (GuOS), des galacto-oligosaccharides (GaOS), du polydextrose, de l'inuline, de la dextrine ou d'une combinaison de ceux-ci.

5. Composition destiné à être utilisé dans le traitement de la polyarthrite rhumatoïde selon l'une quelconque des revendications 1 à 4, dans laquelle les antioxydants correspondent à une combinaison
- d'au moins un antioxydant primaire choisi dans le groupe constitué de la superoxyde dismutase, de la catalase et/ou de la peroxydase, ou d'une combinaison de celles-ci, et
- d'au moins un antioxydant secondaire choisi dans le groupe constitué
des flavonoïdes comme la quercétine et l'épicatéchine susceptible d'être obtenu de *Camellia sinensis* et/ou *Citrus aurantium*,
des polyphénols et de l'anthocyanine susceptible d'être obtenu de *Punica granatum*, *Curcuma longa* et/ou *Crocus sativus*,
de la vitamine A, de la vitamine C, de la vitamine E, des antioxydants polyphénoliques, des caroténoïdes, du glutathion, de la coenzyme Q10, du sélénium, du zinc et/ou du cuivre, ou d'une combinaison de ceux-ci.

6. Composition destiné à être utilisé dans le traitement de la polyarthrite rhumatoïde selon l'une quelconque des revendications 1 à 5, dans laquelle les antioxydants comprennent au moins un acide gras polyinsaturé.

7. Composition destiné à être utilisé dans le traitement de la polyarthrite rhumatoïde selon l'une quelconque des revendications 1 à 6, dans laquelle les antioxydants comprennent un polyphénol, un flavonoïde et/ou une anthocyanine provenant de *Punica granatum*, *Curcuma longa, Citrus aurantium*, *Crocus sativum*, la canneberge, ou une combinaison de ceux-ci.

8. Composition destiné à être utilisé dans le traitement de la polyarthrite rhumatoïde selon l'une quelconque des revendications 1 à 7, dans laquelle les enzymes comprennent, en outre, des enzymes choisies dans les groupes des enzymes protéolytiques et/ou lipolytiques.

9. Composition destiné à être utilisé dans le traitement de la polyarthrite rhumatoïde selon l'une quelconque des revendications 1 à 8, contenant, en outre :
- des vitamines choisies dans le groupe de la vitamine A, de la vitamine C, de la vitamine D, de la vitamine E, de la vitamine B6, de la vitamine B9 et/ou de la vitamine B12 ou d'une combinaison de celles-ci ;
- des oligo-éléments choisis dans le groupe du zinc, du cuivre et/ou du sélénium ou d'une combinaison de ceux-ci ; et/ou
- des acides aminés choisis dans le groupe de la lysine, de la méthionine et/ou de la cystéine ou d'une combinaison de ceux-ci.

10. Composition destiné à être utilisé dans le traitement de la polyarthrite rhumatoïde selon l'une quelconque des revendications 1 à 9, contenant :
20 à 90 % en poids de fibres alimentaires,
1 à 20 % en poids d'antioxydants,
1 à 20 % en poids d'enzymes,
1 à 20 % en poids de vitamines et, éventuellement, d'oligo-éléments,
1 à 40 % en poids d'acides aminés,
pour un total de 100 % en poids de la composition.

11. Composition destiné à être utilisé dans le traitement de la polyarthrite rhumatoïde selon l'une quelconque des revendications 1 à 10, contenant lesdites fibres alimentaires prébiotiques à hauteur de
0 à 30 % en poids et, de préférence, 5 à 15 % en poids de fructo-oligosaccharides,
0 à 30 % en poids et, de préférence, 5 à 20 % en poids de gluco-oligosaccharides,
0 à 40 % en poids et, de préférence, 15 à 30 % en poids de xylo-oligosaccharides,
pour un total de 100 % en poids de la composition.

12. Composition destiné à être utilisé dans le traitement de la polyarthrite rhumatoïde selon l'une quelconque des revendications précédentes, contenant, en outre, des acides gras à chaîne courte associés aux fibres alimentaires prébiotiques.

13. Composition destiné à être utilisé dans le traitement de la polyarthrite rhumatoïde selon l'une quelconque des revendications précédentes, contenant, en outre, 2 à 10 % et, de préférence, environ 4 à 5 % en poids de glutamine, pour un total de 100 % en poids de la composition.

14. Complément alimentaire destiné à être utilisé dans la prévention ou le traitement de la polyarthrite rhumatoïde, contenant une composition selon l'une quelconque des revendications précédentes.

15. Aliment fonctionnel destiné à être utilisé dans la prévention ou le traitement de la polyarthrite rhumatoïde et de la réduction des symptômes de l'ostéoarthrite, contenant une composition selon l'une quelconque des revendications précédentes.
